(19) **Europäisches Patentamt**

**European Patent Office**

**Office européen des brevets**

(11) Publication number : **0 144 936 B2**

(12) # NEW EUROPEAN PATENT SPECIFICATION

(45) Date of publication of the new patent specification : **09.03.94 Bulletin 94/10**

(51) Int. Cl.⁵ : **C07C 51/12,** C07C 53/08,
// B01J23/46

(21) Application number : **84114576.6**

(22) Date of filing : **30.11.84**

(54) **Production of carboxylic acids from alcohols.**

The file contains technical information submitted after the application was filed and not included in this specification

(30) Priority : **02.12.83 US 557274**

(43) Date of publication of application :
**19.06.85 Bulletin 85/25**

(45) Publication of the grant of the patent :
**24.06.87 Bulletin 87/26**

(45) Mention of the opposition decision :
**09.03.94 Bulletin 94/10**

(84) Designated Contracting States :
**DE FR GB IT NL**

(56) References cited :
EP-A- 0 031 606
EP-A- 0 055 618

(56) References cited :
EP-A- 0 097 978
BE-A- 794 803
DD-A- 113 521
DE-A- 2 450 965
DE-C- 3 024 353
GB-A- 1 233 121
US-A- 3 689 533
US-A- 3 845 121

(73) Proprietor : **UNION CARBIDE CORPORATION**
**39 Old Ridgebury Road**
**Danbury Connecticut 06817 (US)**

(72) Inventor : **Schreck, David James**
**5226 Sun Valley Drive**
**Cross Lanes (25313) (US)**

(74) Representative : **Wuesthoff, Franz, Dr.-Ing. et al**
**Wuesthoff & Wuesthoff Patent- und Rechtsanwälte Schweigerstrasse 2**
**D-81541 München (DE)**

EP 0 144 936 B2

## Description

Background of the invention

The production of organic compounds using carbon monoxide or synthesis gas, which is a mixture of carbon monoxide and hydrogen, as reactant has been known for a significant period of time. It is well known that one can produce methanol directly from synthesis gas and that methanol can be further reacted by hydroformylation, homologation and carbonylation reactions to produce acetaldehyde, ethanol and acetic acid or its methyl ester, respectively. It is also known that esters, ethers, and other organic compounds can be reacted with carbon monoxide or synthesis gas to produce oxygenated organic compounds. The difficulties, however, have resided in the ability to carry out any one of these chosen reactions to produce the desired compound at acceptable efficiency, conversion rate and selectivity.

In almost all instances the reaction is generally catalyzed using Group VIII transition metal compound as catalyst and a halogen as the promoter. It is known that many other metal compounds and promoters can be used. In addition, the prior art has disclosed the use of secondary activators or ligands in conjunction with the metal catalysts and promoters. These secondary activators can be other metallic salts or compounds, amines, phosphorus compounds, as well as a multitude of other compounds that have been disclosed in the published literature. Thus, a typical catalyst system contains the metal atom catalyst, promoter and, optionally, ligands, solvents and secondary activators. Though a significant amount of literature does exist describing the production of oxygenated compounds by the isomerization of methyl formate, to our knowledge it does not disclose or suggest our invention. Several of the pertinent patents in this area are discussed below.

FR-A-2 317 269 discloses the production of aliphatic carboxylic acids by the reaction of an alcohol with carbon monoxide in the presence of a catalyst containing at least three essential components, iridium atom, copper atom and halogen.

In EP-A1-0 018 927 there is described a process for the production of monocarboxylic acids by the carbonylation of an alcohol using a nickel catalyst, a halide and a solvent; in this reference synthesis gas is used.

In EP-A1-0 045 637 there is disclosed the direct conversion of formic acid esters to their corresponding carboxylic acids without the presence of carbon monoxide using as catalyst a soluble iridium salt and an iodine promoter.

Another known procedure for producing acetic acid is the catalytic isomerization of methyl formate as shown by the reaction:

$$CH_3OOCH \rightarrow CH_3COOH.$$

This procedure is shown in US-A-1 697 109. The process described is a vapor phase isomerization reaction carried out at 200°C to 450°C at a pressure up to 200 atmospheres using a metal oxide or acetate catalyst.

US-A-2 508 513 claims an iron metal atom based catalyst, e.g. nickel, promoted with methyl iodide for the isomerization of methyl formate to acetic acid, carried out at 300°C to 400°C and a pressure up to 400 atmospheres. Carbon monoxide may be present.

US-A-3 060 233 discloses the carbonylation of methanol to acetic acid using a metal of the iron group of the Periodic Table and a halide. It does not disclose use of rhodium.

US-A-3 769 329 discloses the production of carboxylic acids from alcohols, or the ester, ether and halide derivatives thereof, and carbon monoxide using a rhodium catalyst and a halogen component.

US-A-3 798 267 relates to the conversion of methyl formate to acetic acid in the presence of a catalyst system consisting essentially of activated carbon and a halogen promoter.

US-A-4 194 056 discloses the production of carboxylic acid from methyl formate using a soluble rhodium catalyst, halogen promoter and carbon monoxide.

US-A-4 212 989 describes a process for producing carboxylic acids or their esters by reacting an alcohol or an ether with carbon monoxide using a Group VIII metal catalyst and an iodine promoter.

GB-A-1 286 224 relates to the reaction of methyl formate with carbon monoxide in contact with a rhodium catalyst and a halogen promoter to produce acetic acid.

GB-A-1 293 193 relates to the direct conversion of formic acid esters to the corresponding carboxylic acids, in the presence of carbon monoxide, a catalyst that is a Group IIb or VIII metal and an organic polar solvent.

JP-A1-50-16773 discloses the production of an organic acid from the corresponding formic acid ester in the presence of carbon monoxide using a catalyst system containing cobalt, iron or mercury and a halogen plus an alkali metal salt of a lower aliphatic carboxylic acid, triamine or cyclic amine.

JP-A1-51-65703 discloses the reaction of methyl formate in the presence of carbon monoxide using a system containing a rhenium catalyst and halogen compound to produce acetic acid.

JP-A1-56-22745 discloses the isomerization of a formic acid ester to the corresponding acid in the presence of carbon monoxide, palladium atom, halogen and base.

JP-A1-56-73040 relates to a process for producing acetic acid by isomerizing methyl formate in the presence of carbon monoxide using a nickel catalyst, an iodine compound and an organic nitrogen compound.

JP-A1-56-83439 discloses a method for producing acetic acid by heating methyl formate and carbon monoxide in contact with a catalyst containing palladium, ruthenium and/or iridium metal atom and a halide promoter.

None of the five Japanese Patent Applications disclose a process for producing acetic acid from an alcohol or a formate ester using a catalyst mixture consisting essentially of rhodium metal atom and lithium iodide plus methyl iodide.

From BE-A-794 803 it has been known to produce acetic acid from methanol and CO in the presence of a catalyst system containing a simple rhodium compound, a iodine compound such as methyl iodide, and a further element that, out of 16 useful elements, can also be lithium.

It can be seen that the prior art contains many disclosures dealing with the catalytic production of acetic acid. The art also discloses the production of other organic carboxylic acids by other methods. One of the disadvantages in many of these reactions is the presence of water with the eventual need to remove it from the desired organic acid product. This removal is both complicated and costly. Other disadvantages often include the simultaneous occurrence of other reactions leading to the formation of by-products, such as, dimethyl acetal, methyl acetate, ethanol, etc. These reactions compete with the organic acid production resulting in low conversion rate and selectivity to organic acid.

Many processes employed for the production of organic acids use a catalyst system containing a source of metal atom and a source of halide atom. The alkali metal halides are often mentioned as suitable halide sources, but no distinction is made between any specific one of the alkali metal halides or between any other halogen compound. Nor do any of the references suggest or recognize the synergistic advantage of the use of mixtures of lithium iodide and methyl iodide in conjunction with rhodium catalyst.

Summary of the invention

A process for the production of acetic acid at high efficiency, selectivity and conversion rate by the reaction of mixtures of methanol, methyl acetate and carbon monoxide has been found. The catalyst system charged to the reactor in the process according to the invention contains rhodium or a rhodium compound, lithium iodide and methyl iodide and optionally an 30 organic ligand. The use of mixtures of lithium iodide and methyl iodide in this system within the ranges defined results in a synergistic effect with unexpectedly high efficiency, high conversion rate or activity and high selectivity not heretofore achieved.

Description of the invention

In the catalytic reactions of synthesis gas or carbon monoxide in processes to produce oxygenated organic compounds there are several criteria required of the catalyst. The catalyst must be as stable as possible, it should have a high activity or conversion rate, and it should have as high a selectivity for the desired product as possible.

Stability of the catalyst relates to how long the catalyst remains functional before either breaking down or losing its catalytic effect.

Activity or conversion rate relates to the amounts of reactants the catalyst converts to product per unit of time, generally expressed in g. mole per liter per hour (g mole/l · h).

Selectivity relates to the quantity of desired product produced, generally expressed in mole percent, based on the total amount of both desired products and undesired products produced.

The goal to be achieved is high values for all three criteria and continued efforts are being made to find new catalyst compositions to reach this goal without having a significant detrimental effect on the overall process. Toward this goal the prior art has developed catalyst systems containing a wide variety of metal atoms, promoters and activators, in many cases with diverse other components added. Though these catalyst systems are effective, improvement is always desirable.

The present invention is based on the unexpected and unpredictable discovery that the rhodium-lithium iodide plus methyl iodide system in conjunction with methyl acetate is an unexpectedly superior catalytic system showing a synergistic effect for the production of acetic acid from methanol at unexpected high efficiency, selectivity and conversion rate. It was also found that a ligand, $ER''_3$, can also be present as an optional component of the system. This unexpected synergistic improvement in efficiency, selectivity and conversion rate is achieved when the rhodium catalyzed system's components are maintained within a defined range and when both lithium iodide plus methyl iodide are present as the source of the halogen component in the system. Optionally a solvent and/or diluent can also be present. The improved catalyst system of this invention can be portrayed as containing the components $Rh-LiI-CH_3I-ER''_3$, wherein Rh is the rhodium containing compound and $ER''_3$ is optionally present.

In the process of our invention methanol is reacted with carbon monoxide in the presence of added methyl acetate but without added water using a particular catalyst system containing rhodium atoms and both lithium iodide and methyl iodide. This system synergistically produces acetic acid at unexpectedly high efficiency, conversion rate and selectivity, with a minimum of by-products and without the presence of water. The overall reaction that occurs is theoretically:

$$CH_3OH + CO \rightarrow CH_3COOH$$

The rhodium component of the catalyst system can be supplied from any number of sources, many of these are known to those of ordinary skill in the art. Thus, it is not necessary for an understanding thereof to specifically enumerate every suitable type and every specific compound since any of the known rhodium compounds can be used.

The essential rhodium component of the catalyst system of the present invention may be provided by introducing into the reaction zone a compound of rhodium or may be provided by introducing into the reaction zone rhodium. Among the materials which may be charged to the reaction zone to provide the rhodium component of the catalyst system of the present invention are rhodium metal, rhodium salts and oxides, organo rhodium compounds, and coordination compounds of rhodium.

Specific examples of materials capable of providing the rhodium constituent of the catalyst system of the present invention may be taken from the following list of suitable materials.

Materials.
$RhCl_2$
$RhBr_3$
$RhI_2$
$RhCl_3 3H_2O$
$RhBr_3 3H_2O$
$Rh_2(CO)_4Cl_2$
$Rh_2(CO)_4Br_2$
$Rh_2(CO)_4I_2$
$Rh_2(CO)_8$
$Rh[(C_6H_5)_3P]_2(CO)I$
$Rh[(C_6H_5)_3P]_2(CO)Cl$
Rh metal
$Rh(NO_3)_3$
$RhCl[(C_6H_5)_3P]_2(CH_3I)_2$
$Rh(SnCl_3)[(C_6H_5)_3P]_2$
$RhCl(CO)[(C_6H_5)_3As]_2$
$RhI(CO)[(C_6H_5)_3Sb]_2$
$[(n-C_4H_9)_4N][Rh(CO)_2X_2]$ where X=Cl-, Br-, I-
$[(n-C_4H_9)_4AS]_2[Rh(CO)_2Y_4]$ where X=Br-, I-
$[(n-C_4H_9)_4P][Rh(CO)I_4]$
$Rh[(C_6H_5)_3P]_2(CO)Br$
$Rh[(n-C_4H_9)_3P]_2(CO)Br$
$Rh[(n-C_4H_9)_3P]_2(CO)I$
$RhBr[(C_6H_5)_3P]_3$
$RhI[(C_6H_5)_3P]_3$
$RhCl[(C_6H_5)_3P]_2$
$RhCl[(C_6H_5)_3P]_3H_2$
$[(C_6H_5)_3P]_3Rh(CO)H$
$Rh_2O_3$
$[Rh(C_3H_4)_2Cl]_2$
$K_4Rh_2Cl_2(SnCl_2)_4$
$K_4Rh_2Br_2(SnBr_3)_4$
$K_4Rh_2I_2(SnI_2)_4$

The rhodium concentration can vary over a wide range. Enough rhodium must be present to achieve reasonable reaction rates; however, an excess may on occasion result in undesired by-products formation. The mole ratio of rhodium to alcohol can vary from 1:25 to 1:4,000, the preferred range is from 1:40 to 1:2,000, with the most preferred range being from 1:100 to 1:1,000. The amount used is not a critical feature in this invention and higher rhodium concentrations are acceptable but are influenced by economic considerations.

The second component of the catalyst system is lithium iodide. It can be charged directly, or it can be formed in situ by any combination of lithium compound and iodine component that will result in the formation of lithium iodide during the reaction. The presence of lithium iodide in conjunction with methyl iodide is a critical feature of this invention. Direct charge of lithium iodide is the preferred form. However, any convenient combination of compounds for in situ formation of lithium iodide can be used. This includes the use of lithium carboxylates, carbonates and the like with a halogen compound such as iodine or an alkyl halide. A suitable combination for in situ formation is lithium carboxylate and an alkyl halide.

The third essential component of the system is methyl iodide, which can be added directly or formed in situ by the use of hydrogen iodide, which reacts to produce methyl iodide. The $Rh:Cl_3I$ mole ratio can vary from 1:1 to 1:1,000, preferably from 1:2 to 1:450, and most preferably from 1:8 to 1:150.

Sufficient lithium iodide and methyl iodide must be present to exert a promoting effect on the reaction and to result in high efficiency, conversion rate and selectivity to the corresponding organic acid. The mole ratio of Rh:LiI can vary over a wide range. A Rh:LiI mole ratio of from 1:1 to 1:1000 can be employed, the preferred range is from about 1:2 to 1:450 and most preferably it is from about 1:8 to 1:150. The mole ratio of LiI to $CH_3I$ can vary from 1:1,000 to 1,000:1, preferably from 1:450 to 450:1, and most preferably from 1:150 to 150:1.

As indicated, an organic ligand of the general formula $ER''_3$ can optionally be present in the reaction system. The use of such ligands is known, as are their identities, to those skilled in this art. In this formula E represents a Group VA element, e.g., N, P, As, Sb and Bi, and R'' represents an organic moiety. The ligand can serve as a catalyst stabilizer and/or to further enhance efficiency, conversion rate and selectivity, especially when the reaction is carried out at higher temperatures, for example at about 200°C or above. The ligand also serves to inhibit equipment corrosion in some instances. However, the use of a ligand is not mandatory and the reaction can be carried out without it.

A large number of organic ligands is known and any of these can be used provided they do not have an adverse effect on the reaction. Among those of particular utility are the tertiary amines and the tri- and pentavalent phosphorus compounds. Though those skilled in the art know these compounds, illustrative of suitable compounds one can mention triethylphosphine, tributylphosphine, tri-2-ethylhexylphosphine, triphenylphosphine, tri(4-methoxyphenyl)phosphine, tri-p-tolylphosphine, tri(3-chlorophenyl)phosphine, diphenylhexylphosphine, dimethyl(3-methoxyphenyl)phosphine, dibutyl stearylphosphine, tribenzylphosphine, dipropyl phenylphosphine, ethyl dipropylphosphine, tricyclohexylphosphine, cyclohexyl dibutylphosphine, propyl diphenylphosphine, dipropyl phenylphosphine, phenyl diethylphosphine, tridecylphosphine, trioctadecylphosphine, tribenzylphosphine, methyl diethylphosphine, ethyl diphenylphosphine, tolyl diethylphosphine, cyclohexyl diethylphosphine, diethyl cyclohexylphosphine, bis-(diphenylphosphino)ethane, bis-(diethylphosphino)-propane, bis-(diphenylphosphinol-butane, bis-(diethylphosphino)-octane, trimethylamine, triethylamine, tri-n-butylamine, tri-t-butylamine, tri-2-ethylhexylamine, methyl dibutylamine, tridodecylamine, tristearylamine, ethyl dibutylamine, tricyclohexylamine, triphenylamine, tri(4-methoxyphenyl)amine, tri(pchlorophenyl)-amine, dibutyl phenylamine, dipentyl cyclopentylamine, ethyl diphenylamine, trinaphthylamine, tri-p-tolylamine, tribenzylamine, tri(3-methylcyclohexyl)amine, and the arsines, stibines and bismuthines corresponding to the above-identified phosphines and amines. They can be used singly or, if one desires, mixtures containing two or more ligands can be used. One can also employ a phosphine oxide or phosphite corresponding to the above phosphines as the ligand; these are also well known.

The concentration of ligand charged can vary from a molar ratio of ligand to rhodium of from 50:1 to 1:50, preferably from 10:1 to 1:10, most preferably 3:1 to 1:1.

In addition to the ligand one can optionally have a solvent present. Many essentially inert solvents are known as useful, essentially inert, diluents and illustrative thereof one can mention 1,4-dioxane, the polyethylene glycol di-ethers or esters, diphenyl ether, sulfolane, toluene, carboxylic acids as well as any other diluent or solvent which does not interfere with the reaction to any significant extent. The reaction is preferably carried out in the absence of any solvent or diluent other than those required to introduce reactants or catalyst components.

The present invention does not require the use of acidic halogen promoters, it employs the alkali metal halide lithium iodide. Nor does it require the presence of water or use of large quantities of methyl iodide to give high selectivity to acetic acid, as are taught in U.S. 3,769,329. It was surprising, unexpected and unpredictable that a basic iodide, lithium iodide, would convert methanol to acetic acid because Example 15 of U.S. 3,769,329 taught that use of rhodium, water, acetic acid, methanol and the basic iodide potassium iodide showed little, if any, reaction at carbon monoxide pressure of 55.2 bar (800 psig) and a reaction temperature of 175°C. In this reference, reaction was not observed until the reaction mixture was acidified with a mineral acid, e.g. phosphoric acid. In our invention addition of acid is not required and the basic iodide lithium iodide is used. Under essentially the same conditions of pressure and temperature, a reaction mixture of methanol, methyl acetate, rhodium and lithium iodide produced acetic acid at excellent rates and selectivities; contra to the teachings of U.S.-A-3 769 329.

Also essential to this invention is the presence in the reaction mixture of methyl acetate.

The presence of said compound is critically important.

Thus, it was observed in the reaction to produce acetic acid from methanol that pure methanol in the absence of methyl acetate did not react with carbon monoxide in the presence of rhodium atom and lithium iodide at 55.2 bar (800 psig) and about 180°C. Nor did reaction occur when 1,4-dioxane was used as the solvent. However, the addition of methyl acetate to the reaction mixture resulted in good conversion rates and high selectivity to acetic acid as shown in our examples.

The reaction is carried out at a temperature of from 50°C to 350°C, preferably from 120°C to 220°C and most preferably from 150°C to 200°C. When the reaction is carried out at temperatures above 200°C in the presence of an ER"$_3$ ligand, the phosphines are the preferred ligands.

The pressure of the reaction can be from 10.35 to 690 bar (150 psig to 10,000 psig), preferably from 13.8 to 138 bar (200 psig to 2,000 psig), most preferably from 34.5 to 69 bar (500 psig to 1,000 psig).

The reaction time varies depending upon the reaction parameters, reactor size and charge, and the individual components employed at the specific process conditions. The reaction can be a batch or continuous reaction.

The synergistic effect of mixtures of lithium iodide and methyl iodide on conversion rate was completely unexpected and unpredictable. Significant rate increases were obtained as compared to the use of rhodium with lithium iodide alone or rhodium with methyl iodide alone. The significantly enhanced reaction rates in the production of organic acids from alcohols in the presence of an ester or ester-forming compound are very advantageous in that they result in increased productivity from an available reactor, or they would allow for a significant reduction in size for a new reactor. Another advantage is that equivalent productivity can be achieved with the use of much less of the expensive rhodium catalyst. Use of the system of this invention results in production of acetic acid from methanol at typical conversion rates of from 5 to 7.5 gmoles/l · h and typical selectivities of from 95% to 99% at 180°C and 34.5 bar (500 psig) CO pressure. The values obtained exceed those obtained when either methyl iodide or lithium iodide were used individually with rhodium.

The experiments and examples detailed below were carried out in a Hasteloy® steel autoclave reactor having a volume of 300 ml, which was equipped with temperature and pressure sensing means, heating and cooling means, agitator and inlet and outlet means for introducing and removing components from the reactor. The autoclaves used in synthesis gas reactions are well known in the an and can be used in this process.

Prior to charging the reactants the autoclave was washed with methanol at 100°C under a nitrogen gas pressure of 34.5 to 69 bar (500 to 1,000 psig) by agitating for 30 minutes. The autoclave was drained, rinsed with dry acetone, and dried with nitrogen. The liquid components were charged to the cleaned autoclave first and then the solid components were added and stirred. The autoclave was closed and purged with carbon monoxide and then pressurized to the desired pressure with carbon monoxide. The autoclave contents were heated to the selected temperature, with agitation (usually 750 rpm), in about 45 minutes. After the desired temperature was reached, the reaction was allowed to consume carbon monoxide for the time period indicated. During this period the pressure was maintained by addition of carbon monoxide as needed.

At the end of the reactor run, the contents were cooled, generally to about 10°C. A vapor phase sample was taken for gas chromatography analysis; the gas phase was vented through 2 dry-ice acetone traps and then through a 10 liter saturated solution of calcium hypochlorite to remove metal carbonyls, if formed. The reactor was pressurized three times with nitrogen 6.2 bar (90 psig), and vented through the same system.

The residual reactor contents were dumped into a chilled pressure bottle and sealed. Subsequent analysis was performed using a Hewlett-Packard Model 5880 gas chromatograph equipped with a 3,2 mm (one-eighth inch) diameter by 3 m (ten feet) long column packed with Chromosorb 101.

The following examples serve to further illustrate this invention. In the examples the term "AcAc" means "acetylacetonate". Values given for acetic acid obtained include acetic acid equivalents present as methyl acetate.

Control Experiment A

In this experiment the system contained rhodium atom and lithium iodide only.

The autoclave was charged with 2.06 g of $Rh(CO)_2AcAc$ (8 mmoles), 8.57 g of lithium iodide (64 mmoles), 50 ml. of methylanol (1.25 moles) and 100 ml of methyl acetate. Following the procedure described above the reaction was carried out at 180°C and a carbon monoxide pressure of 69 bar (1,000 psig) for 5 hours. The major product was 1.25 moles of acetic acid. The calculated rate to acetic acid was 2.4 gmole/l · h and the conversion of methanol to acetic acid was 100% at the time the reaction was arbitrarily terminated.

Control Experiment B

In this experiment the system contained rhodium atom and methyl iodide only.

The autoclave was charged with 2.1 g of $Rh(CO_2)AcAc$ (8 mmoles), 9.1 g of methyl iodide (64 mmoles), 50 ml of methanol and 100 ml of methyl acetate and reacted at 55.2 bar (800 psig) in the same manner as Control Experiment A for 4 hours. Major product was 0.49 mole of acetic acid. The calculated rate to acetic acid was 0.86 gmole/l · h and the conversion of methanol to acetic acid was 40% at the time the reaction was arbitrarily terminated.

Example 1

In this example, illustrative of the invention, the autoclave was charged with 1.1 g of $Rh(CO)_2AcAc$ (4 mmoles), 4.2 g of lithium iodide (32 mmoles), 4.6 g of methyl iodide (32 mmoles), 50 ml of methanol (1.25 moles) and 100 ml of methyl acetate. The procedure followed was the same as described in Control Experiment A at a pressure of 41.4 bar (600 psig). after 3 hours all of the methanol had been converted to acetic acid. The calculated rate to acetic acid was 2.2 gmole/l · h at the time the reaction was arbitrarily terminated and the conversion of methanol to acetic acid was 100%.

Comparison to Control A shows significantly lower Rh concentration required to achieve the same results; comparison to Control B shows significantly better reaction rate and conversion.

**Claims**

1. A process for the production of acetic acid which comprises the catalytic reaction of methanol and carbon monoxide in contact with methyl acetate and a homogeneous catalyst system consisting of rhodium or a rhodium compound and a promoter characterized in that the promoter is a mixture of lithium iodide and methyl iodide in a mole ratio of Rh:LiI from 1:1 to 1:1,000 and a mole ratio of LiI:$CH_3$I from 1:1,000 to 1,000:1 and further characterized in, that methyl acetate, but no water, is added to the reaction mixture.

2.  The process as claimed in claim 1 wherein the temperature is from 50°C to 350°C and the pressure is from 10,35 to 690 bar.

3.  The process as claimed in claim 1 or 2 wherein the mole ratio of Rh:LiI is from 1:8 to 1:150.

4.  The process as claimed in claims 1 to 3 wherein a mole ratio of LiI:CH$_3$I is from 1:450 to 450:1 or preferably from 1:150 to 150:1.

5.  The process as claimed in claims 1 to 4 wherein an organic ligand of the formula ER$_3$" is present, wherein E is nitrogen, phosphorus, arsenic, antimony and bismuth and R" is an organic moiety.

6.  The process as claimed in claim 5 wherein the ligand is a phosphine.


**Patentansprüche**

1.  Verfahren zur Herstellung von Essigsäure durch katalytische Umsetzung von Methanol und Kohlenstoffmonoxid in Kontakt mit Methylacetat und einem homogenen Katalysatorsystem aus Rhodium oder einer Rhodiumverbindung und einem Promotor, dadurch **gekennzeichnet**, daß der Promotor ein Gemisch aus Lithiumiodid und Methyliodid in einem Molverhältnis Rh : LiI von 1 : 1 bis 1 : 1000 und einem Molverhältnis von LiI : Ch$_3$I von 1 : 1000 bis 1000 : 1 ist und welches weiterhin dadurch gekennzeichnet ist, daß Methylacetat aber kein Wasser der Reaktionsmischung zugegeben wird.

2.  Verfahren nach Anspruch 1, bei dem man bei 50 bis 350°C unter einem Druck von 10,35 bis 690 bar arbeitet.

3.  Verfahren nach Anspruch 1 oder 2, worin das Molverhältnis Rh : LiI zwischen 1 : 8 und 1 : 150 liegt.

4.  Verfahren nach einem der Ansprüche 1 bis 3, worin das Molverhältnis LiI : CH$_3$I zwischen 1 : 450 bis 450 : 1 oder vorzugsweise zwischen 1 : 150 bis 150 : 1 liegt.

5.  Verfahren nach einem der Ansprüche 1 bis 4, worin ein organischer Ligand der Formel ER$_3$" vorhanden ist, worin E Stickstoff, Phosphor, Arsen, Antimon und Bismut und R" für eine organische Gruppe steht.

6.  Verfahren nach Anspruch 5, worin der Ligand ein Phosphin ist.


**Revendications**

1.  Procédé de production d'acide acétique qui comprend la réaction catalytique de méthanol et d'oxyde de carbone au contact d'acétate de méthyle et d'un système catalyseur homogène formé de rhodium ou d'un composé de rhodium et d'un promoteur, caractérisé en ce que le promoteur est un mélange d'iodure de lithium et d'iodure de méthyle dans un rapport molaire Rh:LiI de 1:1 à 1:1000 et un rapport molaire LiI:CH$_3$I de 1:1000 à 1000:1 et caractérisé en outre en ce que de l'acétate de méthyle mais non de l'eau est ajouté au mélange réactionnel.

2.  Procédé suivant la revendication 1, dans lequel la température va de 50 à 350°C et la pression va de 10,35 à 690 bars.

3.  Procédé suivant la revendication 1 ou 2, dans lequel le rapport molaire Rh:LiI a une valeur de 1:8 à 1:150.

4.  Procédé suivant les revendications 1 à 3, dans lequel le rapport molaire LiI:CH$_3$I va de 1:450 à 450:1 ou de préférence de 1:150 à 150:1.

5.  Procédé suivant les revendications 1 à 4, dans lequel un ligand organique de formule ER$_3$" est présent, E désignant l'azote, le phosphore, l'arsenic, l'antimoine et le bismuth et R" est un groupement organique.

6.  Procédé suivant la revendication 5, dans lequel le ligand est une phosphine.